# EUROPEAN PATENT APPLICATION

(11) **EP 1 364 679 A2**
(43) Date of publication of application: **26.11.2003**
(21) Application number: 03380110.1
(22) Date of filing: 08.05.2003
(51) Int. Cl.: A61N 2/02

(54) **Device for generating multiple magnetic fields used in magnetotherapy, and magneto acupuncture**

(30) Priority: 21.05.2002 ES 200201159
(71) Applicant: de la Cal, Antonio Madronero, 20007 Madrid (ES)
(72) Inventor: de la Cal, Antonio Madronero, 20007 Madrid (ES)

(57) **Abstract**

The present invention is basically constituted by an electronic power supply [1] that produces different types of currents that feed the corresponding electrically independient coils housed in a reel, mounted together in the applicator [2], so that the resulting magnetic field is the sum of the superimposed fields produced in each coil. A set of magnetic fields with different frequencies is simultaneously generated in the applicator. The therapeutic effects of the compound magnetic field are different from those obtained from the one frequency magnetic fields usually used in magnetotherapy. An optional display [3] is comprised in the generator [1] and by means of a switch (not shown in the figure), a choice of the parameters of the currents generated by the independent generator units is possible. These parameters are adjusted by means of the controls of the knobs [4] (frequency) and [5] (intensity). The number of these pairs of controls (there are three in the figure) can be two or more. The corresponding generated currents, starting from the pairs of plugs [6] and [7] are transmitted by the cables [8] and [9] to each one of the coils [10]. The fields of the coils are therefore mixed, constituting in this way the mentioned applicator [2]. A nucleus of material ferro or ferrimagnetic could be included in the applicator, as well as magnetic disks surrounding the tip of the applicator's nucleus.

## Description

### Introduction

A review carried out by the inventor, Gálvez Fraide and other [J. M. Gálvez Failde, A. Madroñero de la Cal, M. Valls Cabrero, R. Camera Anguita, M. Miranda Mayordomo and J. M. Gálvez Hernández. "Magnetoterapia: bases físicas y biológicas de su aplicación terapéutica " *Rehabilitación*. 24, 1 (3-9), 1990], shows that Magnetotherapy was already applied in the Antiquity. Pliny the Young, in their "Medical matter" (77-78 d. of C.) it recommended the use of the magnetic stone for ocular and urinals, treatments, vaginal flow, haemoptysis, ulcers and burns. In the already entered the XVIII century, in 1775, Bolten writes, in Hamburg, his treaty of Eine Nachricht von einem mit dem künstlichen Magneten gemachten Versuch in einer Nervenkrankheit (Communication of an investigation carried out with artificial imams for treatment of a nervous illness), and in 1776, Elisha Perkins patents her metallic "Apparatus of Perkins for treatment of the pain", apparatus with which George Washington was treated, although the application of magnetic fields by means of solenoids doesn't take place up to 1869, for Gaylord Wilshire.

In the seventies, already in the XX century, a R. Lechiner and R. Ascherla's paper entitled "Bases and clinic study on the therapy for electrodynamics fields that don't affect to the processes of bone repair" [R. Lechiner and R. Ascherla "Grundlagen und Klinik der elektrodynamischen Feldtherapie bei Knochenheilungsstörungen". *Med. Orthop. Technik*, 2, pp. 43-49, 1978] was published. The authors successfully used fields of low frequency on soft tissue, with analgesic effects, increase of the blood irrigation and activation of the cellular processes. However the studies on Magnetotherapy become more rigorous since the spreading of its application for the stimulation of bone repair processes ("magnetosteogenics"), using fields of higher frequency that stimulate the pseudoartrosis resolution and of consolidation in delayed unions [to see, for example, A. Madroñero de la Cal and P. Guillén García. "Magnetoterapia and magnetosteogenia". *Clínica Médica* (Barcelona), 85, pp. 838-839, 1985].

### Invention object

The object of the present invention is a generator of compound magnetic fields having therapeutic capability, due to the fact that such magnetic fields are applied with a proper frequency range by means of a transducer. This is constituted by several electrically independent coils. All coils receive proper currents, also independent, so that an odd composite magnetic field having specific therapeutic applications is generated.

### Definitions.

In order to avoid all confusion in the terms that are used when describing the present invention, we insert here the sense that one gives when using them next.

*Electric Currents Supply (ECS)*. It is a part of the present invention that allows to generate two or more electric currents of different type to feed the coils where each component of the therapeutic magnetic field is generated. Using conventional electronic technology, this generator can produce direct or alternating currents in their diverse modalities, according to the required characteristics of a magnetic field looking to each therapeutic requirement. Nevertheless, in some applications of the present invention, it is possible to get some component of the therapeutic composite magnetic field by using permanent magnets, instead of a coil powered by a direct current supplied by ECS.

*Coils*. In the present document, a coil is a set of one or more electrically conductive wire turns fed by one of the currents produced by ECS. Each one of these coils or group of wire turns, are electrically independent. After being connected to ECS, it produces a magnetic field component. Each coil can be applied on different anatomic portions or grouped in a frame or reel. Such reel can be applied on an area of the body in order to produce simultaneous therapeutic effects with accumulative biological/physiological actions.

*Applicator*, It is an essential part of the present invention, a frame or device that contains the reels or coils that generate the composite magnetic fields. This part of the device can be or not, according to the details of each particular use, a unique element having a single transducer connected to ECS. It can as well be constituted for autonomous units connected to ECS by an independent electric cable for each unit. The term "applicator" is used because it is the part of the device object of the present invention that is applied to each patient to get the therapeutic effect of the magnetic fields generated in a single or various parts transducer.

*Punctor*. It is a cylindrical applicator constituted by a magnetic material ended in a blunt tip, and surrounded by a coil. In order to apply the treatment, the therapist should put the punctor into some active acupuncture point of the patient's body.

*Perforated disk or "washer"*. Cylindrical or prismatic body whose base is very big in comparison with its height, with a central hole. It usually constitutes an accessory for the punctor, the above described device object of the present invention. It can as well to be considered as a significant part of it. The disk can be formed by or contain one or several permanent magnets; their set is, in that case, as sectors in a washer with a circular row. The perforated disk can as well be a reel with a central hole containing one or several independent coils powered by one or several currents supplied by the ECS.

*Intensifier*. Accessory of the applicator made up by one or several disks that surrounds the tip of the punctor. The following are a few concepts related to magnetism that we find useful to reproduce.

*Direct current*. Electric current in which the flowing of electrons always moves in the same direction, although the intensity can be variable. The generator object of the present invention can generate a pure continuous current (unvarying intensity), or follow a discontinuous variation in time (current pulses) having wave different wave form such as squares, triangles, etc.

*Alternating current*. This is an electric current in which the flow of electrons changes its direction periodically. The generator object of the present invention can therefore generate an average pure sinusoidal, pulses of constant or decreasing strength, etc.

*Current frequency*. This term refers to the number of times that a periodic process repeats for unit of time. The direct current can also be considered an alternating current of zero frequency.

*Magnetic field*. The region in the nearby of permanent magnets or conductor wires run by electric current, in which magnetic forces can be detected.

*Diamagnetic*. This term is applied to a material that has a minor magnetic permeability that the vacuum, and is repelled by the action of a powerful magnet.

*Paramagnetic*. This term is applied to a material that has larger magnetic permeability that the vacuum and is softly attracted by the magnets.

*Ferromagnetic*. This term is applied to materials that, as the iron, have very high magnetic permeability; they can be saturated and permanently magnetised. The characteristic of the ferromagnetic materials is that the value of the permeability depends on the intensity of the magnetic field. Typical examples of ferromagnetic materials are, besides the iron, the nickel and the cobalt.

*Ferrimagnetic*. This term is applied to materials, usually ceramic, known industrially as "ferrites" or "electroceramics". They become spontaneously magnetized to ambient temperature in nature, without needing an artificial magnetization to act as permanent magnets. The materials typically known as ferrimagnetics are metallic double oxides (oxides of two different metals), as for instance the ferrites of Fe and Mn and those of Fe and Zn.

### Brief summary of the invention

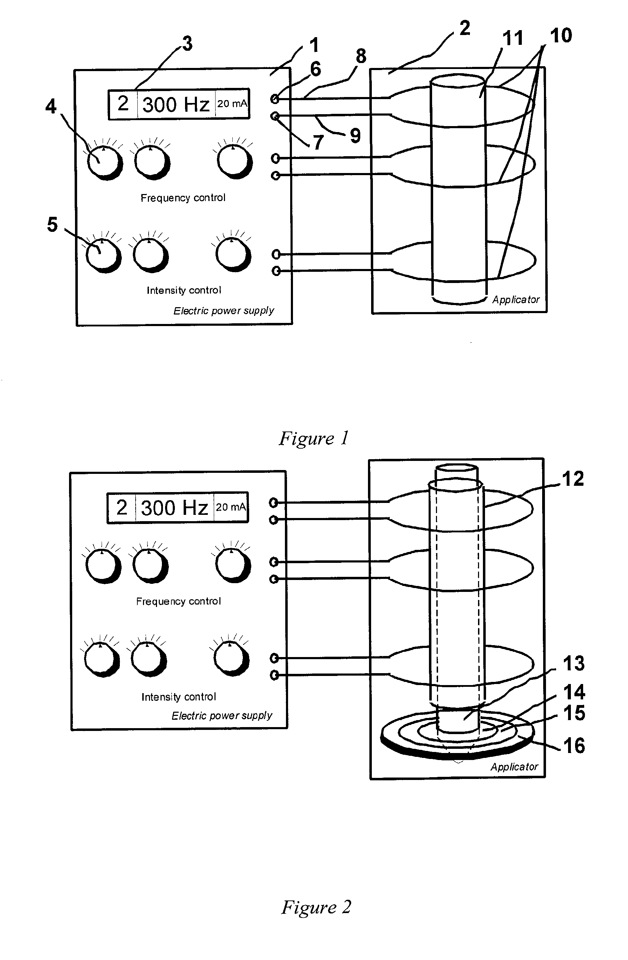

Essentially, the present invention contains (see figure 1) an electric current supply *ECS* [1] that contains two or more independent sources of currents that feed the corresponding coils, also electrically independent, joined together or combined in the applicator [2], so that the magnetic field generated are mixed or superimposed. Due to this electrical separation and according to the clinical demand of each problem, it is possible to run currents with different frequencies in each coil. The goal of the applicator is to procure the superimposition of such fields, reaching their addition into the anatomical portion that is being treated.

The application of a complex magnetic field composed of different frequencies instead of a unique field with a unique frequency, is because that a complex field produces different therapeutic effects to those originated by an one frequency field, as it is generally described in the bibliography.

The essential of this invention is that although it uses well-known frequencies published by several authors, it achieves greater levels of efficiency due to a synergetic effect. The association of therapeutic effects is positive and there are not records of undesirable association.

Of course, the continuous field that is usually produced from a direct current of constant intensity flowing for a coil can be achieved, for commodity reasons in some cases, from a permanent magnet instead of the coil.

The ECS (Electric Currents Supply) [1] optionally comprises a display [3] to show the figures of the parameters of each power supply units. These parameters are adjusted by means of the potentiometer knobs [4] (frequency) and [5] (intensity). The number of these couples of controls (three in the figure) can be two, three or more. The corresponding generated currents, starting from the couples of plugs [6] and [7], are transmitted by the cables [8] and [9] to each one of the coils [10] located in the applicator [2], around the support [11]. It is as well possible to include a nucleus of ferromagnetic or ferrimagnetic material in the applicator.

The support [11] can be cylindrical, as it is shown in figure 1, but it can also be constructed according to any other form of hollow structure where a human member or anatomical portion can be introduced in order to receive a magnetic field. The support works as a proper frame for the addition of the magnetic fields produced in each active coil. Due to their vectorial character, each portion of magnetic field generated in each coils [10] powered by independent currents, is evidently aggregated to generate a compound field. The support allows these coils to be housed with an adequate orientation. Therefore, it is possible to produce a compound field with any orientation according to the requirements of the treatment.

### Description of the Prior Art.

The number of devices for the clinical application of magnetic fields described in the different documents of patents and models of utility is huge. In the following paragraphs, only the devices that use magnetic fields of different frequencies simultaneously are described. With this restriction, the number of patents is substantially smaller. In this way, the following documents can be enumerated:
- WO8905673. Magnetic field generator for therapeutic purposes
- US5718662. Apparatus for the magnetic stimulation of cells or tissue
- WO9955420. An apparatus for the treatment of disorders of tissue and/or the joints.
- EP0100050. Device for an acupuncture-magnetotherapy treatment.
- W00007665. PEMF treatment for osteoporosis and tissue growth stimulation.
- DE3417773. Device for producing pulsating magnetic fields for therapeutic purposes.
- DE3344394. Magnetic field therapy device with vectorial field coils.
- DE3246128. Magnet arrangement for amplifying a magnetic forced field, in particular for therapeutic purposes.
And the following Spanish models of utility:
- U0271816. Generador de campos magnéticos secuenciales, inductores de campos bioeléctricos (Generator of sequential magnetic fields, for induction of bioelectrics fields).
- U0278087. Aplicador de campos magnéticos para podología (Applicator of magnetic fields for podology).
- U0278088. (Aplicador bucal de campos magnéticos para odontología) Oral applicator of magnetic fields for dentistry.
- U8800625. (Generador portátil de campos magnéticos) Portable generator of magnetic fields.
- U8901103. (Suplemento de asiento y respaldo de aplicación de campos magnéticos) Seat and back supplement for application of magnetic fields.

The patent WO8905673, first of the above mentioned, describes a generator that produces a wave of high frequency and simultaneously a second wave of very low frequency that is adjusted for each patient. These waves so generated are taken to an *unique coil* that surrounds a nucleus; the generator sends both waves with alternation to the reel, so that the slowest frequency cut the flow pass or give way the current with higher frequency.

The patent US5718662 uses a power supply that generates currents of different frequencies that, through a capacitor, reach to a *unique coil* following a fixed sequence in time.

The patent WO9955420 uses different coils housed in a support, but the magnetic fields are not superimposed because the device allows to choose only one of *the coils*, the nearest to the organ treated, or several coils in the nearby, therefore generating a magnetic field having a unique chosen frequency.

In the rest of the formerly mentioned patents, only one frequency is used in each treatment, although from the generator allows to generate currents with different frequencies. The different locations of the coils are only choosen looking to the commodity of the patient. (for example, in the patent WO0007665, the coils are located on different parts - seat, back, arm and foot- of an armchair were the patient is seated).

In the present invention, contrary to the formerly mentioned patents, a series of coils (*always more than one)* fed by different currents, is always present.

The coils are housed on a unique frame. Both the coils and the frame constitute a converter of current into magnetic field that is denominated *applicator*, due to the fact that it allows to apply the magneto-therapeutic treatments to human members and organs.

### Brief description of the drawings

### Figure 1. Sketch of the essential parts of the present invention:

[1] Electric current supply ECS, essentially comprised for:
   - [3] Display, optional
   - [4] Knobs of the potentiometers for choice of frequencies
   - [5] Knobs of the potentiometers for regulation of the current intensity for each Frequency.
   - [6] and [7] Output plugs for supplied currents
   - [8] and [9] Cables that, starting from the couples of plugs [6] and [7], transport the currents generated in [1] to each one of the independent coils.
[2] Applicator that contains:
   - [10] Each one of the coils (for further simplicity only one wire whorl is drawn) that are fed by the currents generated in [1].
   - [11] Reel that usually has a cylindrical shape, like it is shown in the figure, on which the coils are wound [10]. Sometimes all of the coils can not be coaxial all of them, as it is shown in the figure for simplicity.

### Figure 2. Simplified sketch of the invention with optional added extras:

The applicator can optionally contain, besides the indispensable elements included in figure 1, such as [10] (each one of the coils that receive the currents generated in [1]) and [11] (reel on which the coils are wound [10]), other items like:
- [12] Support, equivalent to the [11] of figure 1, but hollow in this case
- [13] Ferro- or ferrimagnetic nucleus, having a blunt tip.
- [14] Thin disk with a central hole (to be run through for [13]), made up by a permanent magnet or a coil fed by any type of current, preferably a direct one.
- [15] and [16] one or more concentric optional disks [14], each of them formed by a permanent magnet or a coil fed by any kind of electric current, preferably a direct one.

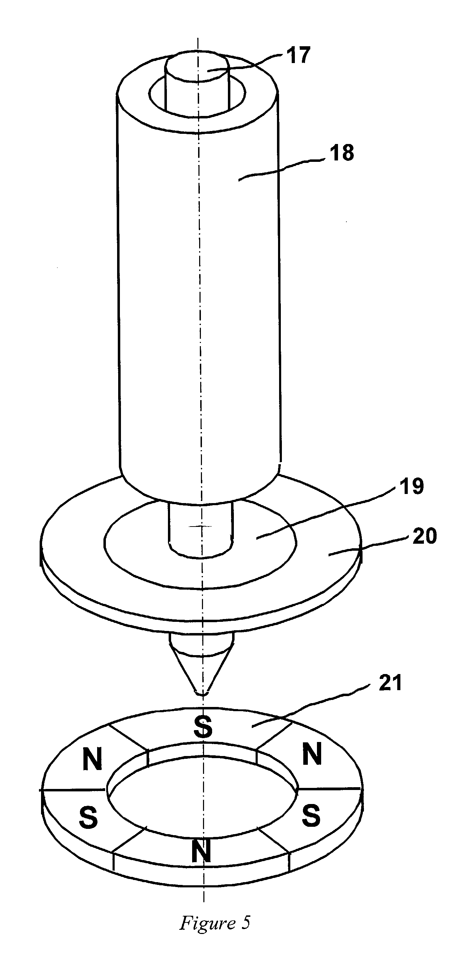

One of the disks can be magnetised so that its surface presents an even number of north and south poles in line or succession so that they constitute a collar plate made up of an even number of juxtaposed poles north south (see [21] of figure 5).

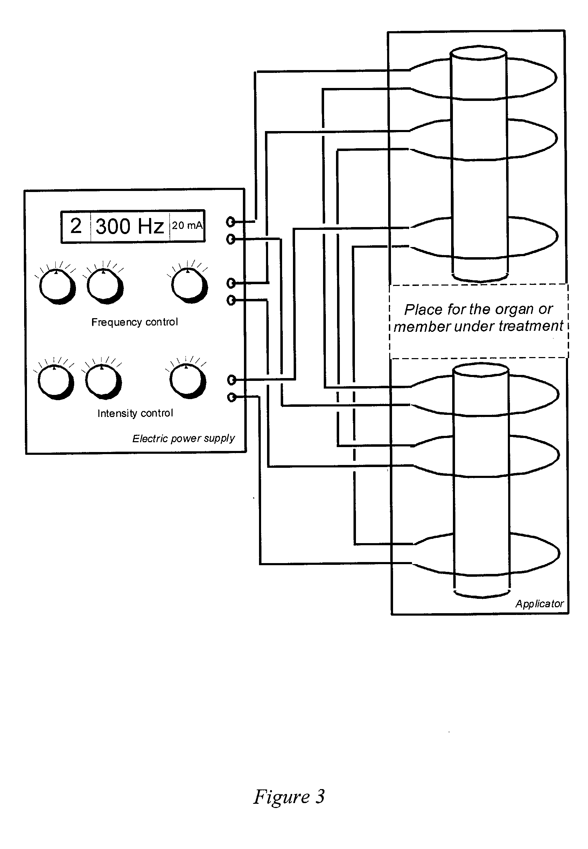

### Figure 3. Variant of the applicator.

For some therapeutic applications, it is convenient to divide the applicator into two symmetrical parts or halves, as it is shown in figure 3. In these cases, each coil is divided in two fractions, housed each one in a half of the applicator and connected electrically. In this way the magnetic field generated by each coil runs through the human organ or member under curative treatment located between both halves of the applicator during the therapeutic session.

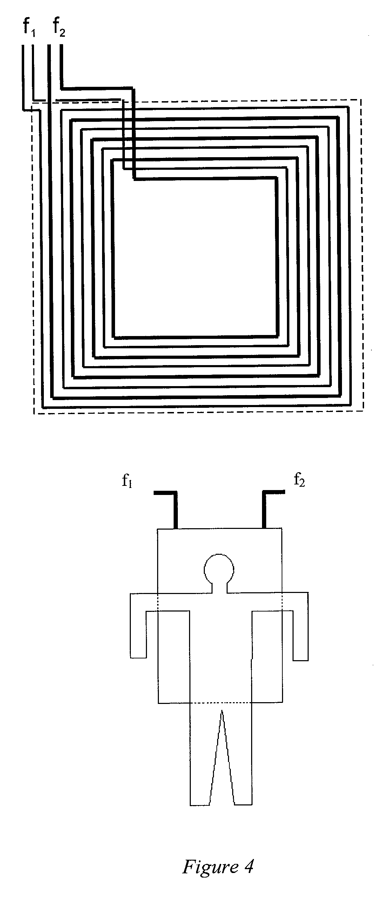

### Figure 4. Magnetic mat

In the upper part, the electric architecture of the double layered flexible mat (represented by the dotted rectangle) that acts as a flexible frame, is sketched. Between both layers, two helical plain coils are housed. Both spirals can be fed by currents of different frequencies. In order to distinguish them, the two coils have been drawn with different lines (continuous and dashed). Below, an outline of the patient's position on the mat is shown. The coils are obviously cased in the mat.

### Figure 5. Applicator for magneto acupuncture.

- [17] Punctor of ferro- or ferrimagnetic material (equivalent to [13] of figure 2).
- [18] Reel formed by one or more independent coils that receive the current (alternating, direct or both) equivalent to the coils [10] of figure 1.
- [19] and [20] permanent magnets or discs of ferro- or ferrimagnetic material (housed in the structure [11] and equivalent to the discs [13], [14],..., of figure 2).
- [21] Collar plate with alternatively north/south permanent magnets. This item can substitute the disc [20].

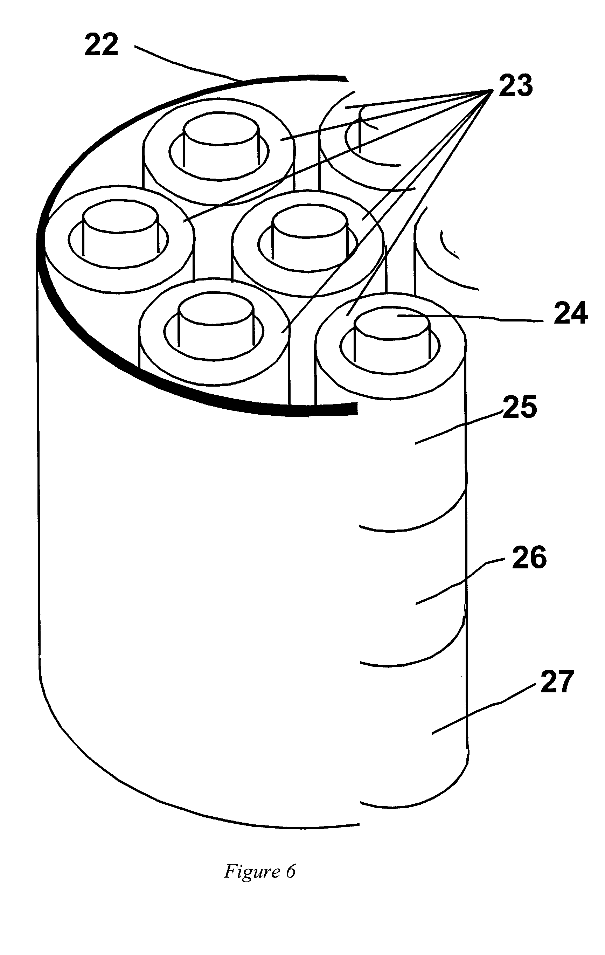

### Figure 6. Multivibrator.

Formed by a shell [22] that contains a sheaf of applicators similar to the single applicator of the figure 5, with or without the disks [19], [20], [21] that can be added outwardly to the shell.
- [23] Windings of each one of the applicators (each one an equivalent one to the [18] of figure 5). Each winding contains at least two coils (in the figure three coils have been drawn: [25], [26] and [27]).
- [24] Ferri- or ferromagnetic nucleus (each one is equivalent to the [17] of figure 5), surrounded by the coils [23].

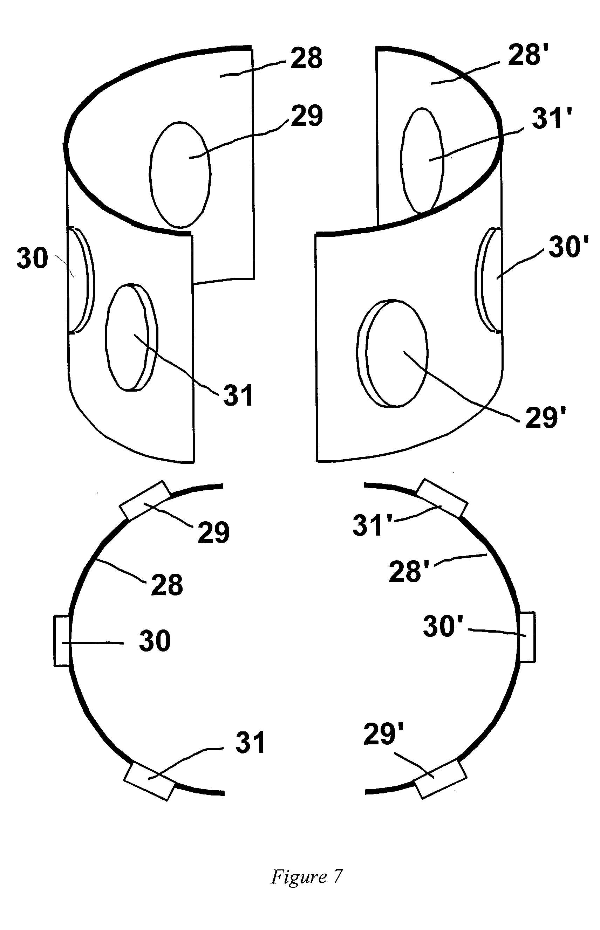

### Figure 7. Applicator of two symmetrical valves.

The support ([11] of the figure 2) is divided into two halves (as it is shown in figure 3). In this case, each one of the coils is divided into two equal halves. Each half faces the other, as indicated in the figure 7, so that the vector of the magnetic field generated by each pair of halves is normal to the axis of the cylinder formed by the two halves joined together. The three fields form angles of 60° between them:
- [28] and [28'] Frame made for the two joined halves.
- [29] and [29'] A pair of half coil, electrically connected.
- [30] and [30'] A pair of half coil, electrically connected.
- [31] and [31'] A pair of half coil, electrically connected.

The electric connection of the two halves is as indicated in figure 3. When the coils are fed with direct current, each half coil has opposite polarity to that of the other half.

### Detailed description of the invention

The reason of the present invention is the possibility of mixing several magnetic fields of different frequencies and orientations, in order to maximize the therapeutic effect resulting from the application of a composite field. It is based on the well-known fact that the electromagnetic fields have certain biological effects that depend on their frequency. Different well-known therapies, such as Radiotherapy, Diapulse and Magnetotherapy are good examples.

With the applicators described in the present invention, it is possible to apply, for example, a field with a certain frequency on an extremity of a patient, so that the direction of the exogenous field is proximal-distal. Simultaneously, it is possible to apply another exogenous magnetic field (with a different frequency) having an internal/external direction. Each field is intended to produce a reactive action-reaction effect.

The essential advantage of this invention is that it makes the simultaneous addition of well known frequencies possible, in those cases in which this addition supposes an additional advantage (positive synergy). For cases in which the association of different frequencies supposes a negative synergy, the addition is avoided.

In order to supply the currents needed to produce magnetic fields of different frequencies, according to the demands of each case, the ECS should be able to produce, for instance:
- One or several direct currents of different intensities but constant in time.
- One or several alternating currents whose intensity varies throughout time following the form of a sinusoidal wave of preset frequency. When several alternating currents of this type are generated, their effective intensity can be different and adjustable for each one.
- Pulses of direct or alternating current. These pulses can have different shapes as squares, triangles or dumped outlines.

The applicators (the equipment can have more than one) [2] are, as it is shown in figure 1, similar to orthopaedic devices in which some windings of metallic wire have been inserted. These windings are fed by the currents produced by the electronic ECS [1]. The situation and orientation of each winding allows to form a complex magnetic field generated jointly by each wiring housed in the reel or frame (it mixes the fields that each singular winding would generate if it worked alone). The anatomical zones or members of the patient are submerged to receive the therapeutical effect of the magnetic field.

Each applicator contains a series of windings [10] that receive currents generated in [1] and a frame or support [11] made of a para- or diamagnetic material that works as a support of the groups of coils [10]; in the figure only a spire has been drawn in each coil. Optionally, the support [11] can be a hollow cylinder, as it is shown in figure 2. A preferably cylindrical bar [12], made of a ferro- or ferromagnetic material, can be placed in it. The tip of the bar can optionally cross through a hole in the centre of a perforated disc [13]. Such disc can be formed by a permanent magnet or by a looped winding, preferably fed by a direct current. The perforated disc [13] can be surrounded by one or more perforated discs [14], [15], etc., formed by permanent magnets or windings shaped as a collar, fed by direct or alternating currents. One of the discs, preferably the outer one, can be formed by several permanent magnets (in even number) inserted in a support such as a neck plate.

Optionally, the frame of the applicator (and the corresponding coils) can be divided into two parts or halves, as it is shown in figure 3, so the magnetic field generated by each half of coil or group of spires can cross through the organ or human tissue that is being treated. Each fraction of a coil is electrically connected to the complementary fraction of the coil attached to the other half.

### Examples

We are now describing different ways of building up an applicator. The enumeration or description of these applicators is not limitative of the present invention that intends to show, only as an example, some ways to carry it out.

### Example 1. Magnetic mat for Magnetotherapy (see figure 4)

It consists in a double layered flexible mat in whose interior there are at least two concentric and independent windings (as it is pointed out in the upper outline of figure 4), so that the resulting magnetic fields are superimposed and have the same direction. It is used in patients lying in decubital position (as it is indicated in the lower sketch at the figure 4).

### Magnetic mat

Above, electric outline of the double layered mat (represented by the dotted rectangle) that acts as a flexible frame. Between both layers, two helical plain coils are housed. Both spirals can be fed by currents of different frequencies. The two coils have been drawn with different lines (continuous and dashed) in order to distinguish them. Below, an outline of the patient's position on the mat is shown. The coils are obviously cased in the mat.

Each coil (whose group corresponds to the "Applicator" [2] of figure 1) is connected independently to one of the sources of the electric current supply ECS ([1] of figure 1). From the magnetic point of view, the two faces of the double layered mat, shall be clearly signalled by the manufacturer, in order to facilitate their distinction by the user. When the patient leans on a labelled surface of the mat, he is receiving a magnetic field with a certain polarity (when one of the coils is fed with direct current). However, it is advisable to use two different alternating currents simultaneously: a lower frequency current (for example 9 Hz) and a higher frequency one (for example 30 Hz).

The typical user of this magnetic pad is a person who wants to alleviate chronic medium pain in the back. Also, menopausic female patients can find the magnetic pad beneficial because it can alleviate their osteoporosis in the lumbar area. In back troubles, the treatment of osteophenia and osteoporosis with this pad not only can stop the course of the illness, but it can cause a remission of the process as well.

### Example 2. Applicator for magneto acupuncture (see figure 5).

The Applicator contains, besides the coil [18], a punctor [17] and one or more (generally only two as it is shown in the figure) perforated magnetic discs ([19] and [20] or [19] and [21]) surrounding the second ([20] or [21], in case of using only two), as it is shown in figure 5 (these discs were mentioned before as [13] and [14] of figure 2).

The punctor [17], a pointed bar of ferromagnetic material, is a cylinder of small diameter (a typical design would have a diameter of 2 mm and a length of 5 to 10 cm) finished in blunt tip that during the use is surrounded by a winding [18] (as the referred as winding [10] at figure 1), with one or several independent coils, powered by the electronic equipment ECS. In each therapeutic session, the tip is put on the acupuncture point where the treatment is applied.

The key tool is the set of the two magnetic discs [19] and [20] or [19] and [21]) labelled as *intensifier*. This group can be a double disk, [19] and [20], with a central hole. There are several possible combinations by interchanging different discs in the setting up of the pieces:
- The disc [19] can be made of a magnetized magnetic material or constituted by a support of diamagnetic material that holds a mounted plane coil, which receives the electric current from the generator ECS.
- The disc [20] can equally be of magnetized magnetic material or of a diamagnetic material with an assembled plane coil on it, receiving the current from the generator ECS.
- The disc [20] can be substituted for another one [21] formed by a series of mounted permanent magnets with alternate polarities. Although in figure 5 the disc [21] contains six magnets, their number can be any, although an even number of these magnets is preferable.

The group of the two disks has a preferable diameter of 2 cm. Whichever the type of generation of the magnetic field in the disc and the collar plate [19] and [20] is (permanent magnets or continuous magnetic field produced by the direct current that feeds the coil), such magnetizing should present different magnetic polarities in both pieces.

The topic of the polarity is important. In therapy with magnetic fields, the north pole of a magnet has a relaxing effect (or "dispersing" in the language of acupuncturists) on the bioelectric energy. Contrarily, the south pole has a stimulant effect on the bioelectric energy, that is to say, it increases the energy in that point.

Therefore, when a relaxing effect is aimed, the winding [18] will be fed with a direct current, taking care that the tip of the ferromagnetic bar [17] acquires a magnetizing with north polarity. Besides this, the face of the centrally perforated disc [19] in contact with the human skin will have a south polarity. Due to this combination, an action- reaction effect is procured. Such effect is similar to the repeated immersion of the skin in hot and cold water.

Similarly, when a stimulant effect is aimed, the feeding of the bar [17] will provide a punctual south polarity in the tip. And it will be surrounded by an intensifier [19] that bears a north polarity in the face that contacts the human skin.

If the disc [19] is set up inside the collar plate [20], both parts will be mounted so that they present opposite polarities in each face, north for the inner disc and south for the outer one, or viceversa.

### Example 3. Applicators type multivibrator (see figure 6).

It is a set or bundle of wirings as [23] (equivalent to the [18] of the figure 5) formed by for two or more coils (in the figure three have been drawn: [25], [26] and [27]) with a nucleus ferro- or ferrimagnetic [24] put together inside the capsule [22], constituting an applicator with capability to use different frequencies simultaneously.

The essential of this type of applicator, is that the fields produced by independent currents with uneven frequencies (generated by the coils [25], [26], [27]) can mix in each nucleus and allow the positive effects of the mixture of fields to take place. A careful choice of the frequencies procures the conjugation of their effects according to the action-reaction or synergy phenomena, already described in the present invention.

Beside this, due to the fact that it is possible to control the different fields that appear in the ends of each bar, it is possible to obtain an action-reaction effect only by using this complex applicator. For example, the nucleus located in the central position can have a field with south polarity, while the surrounding nuclei can have a field with north polarity. From this point of view, it is possible to say that this applicator can be considered a generalization of the applicators described in the example 2.

### Example 4. Applicator of two half cylinder components (see figure 7).

It is an applicator that, from the mechanical point of view, is constituted by two hemicylinders [28] and [28'] that, after being adjoined, constitute a quite cylindrical muff with some degree of cone appearance. In their interior, there are six regularly fixed coils ([29], [29']. [30], [30'], [31] and [31']), interconnected two by two, housed in opposite positions distributed at 60° in the complete cylinder.

The magnetic field that each coil produces is added to the field produced by the opposite coil, located in the other half cylinder support, so that inside the applicator there are three independent magnetic fields, with one frequency each. Consequently, the vectorial sum of these three components can change of direction and strength continuously.

## Claims

1. Device to be used to impart therapeutic treatments with magnetic fields and magneto acupuncture, constituted by a generator that produces currents of uneven frequencies that feed a set of corresponding coils, that apply the magnetic fields generated to any anatomical area chosen to be treated, **characterized** because: a) the generator simultaneously supplies, by independent output plugs, at least two types of electric currents. One or more of these currents can be direct and the rest should be any sort of alternating currents, even intermittent currents, and b) because each one of these currents feeds an electrically independent coil, each coil is mounted on a support that at least allows imposing the direction of the vector that defines the magnetic field generated by such coil.

2. Device to be used to impart therapeutic treatments with magnetic fields and magneto acupuncture, according to claim 1, **characterized** because at least one of the continuous currents supplied by the generator has a preset intensity, constant in time.

3. Device to be used to impart therapeutic treatments with magnetic fields and magneto acupuncture, according to claim 1, **characterized** because the intensity of at least one of the alternating currents supplied by the generator varies throughout time following the form of a sinusoidal wave of a preset frequency and intensity.

4. Device to be used to impart therapeutic treatments with magnetic fields and magneto acupuncture, according to claim 1, **characterized** because at least one of the direct or alternating currents supplied by the generator consists in current pulses.

5. Device to be used to impart therapeutic treatments with magnetic fields and magneto acupuncture, according to claim 1 and at least one of the claims 2, 3 or 4, **characterized** because the support where the coils are housed is constituted by two physically separated similar parts; each part contains a fraction of each coil that is electrically connected to the other fraction of the same coil.

6. Device to be used to impart therapeutic treatments with magnetic fields and magneto acupuncture, according to claim 1 and at least one of the claims 2, 3 or 4, **characterized** because the frame or support in which one or more independent coils forming a unique winding are housed, is a tubular and hollow pipe in whose interior there is, as part of the applicator, a bar of a ferri- or ferromagnetic material finished in a blunt tip. This tip contacts the point of the skin that is being treated.

7. Device to be used to impart therapeutic treatments with magnetic fields and magneto acupuncture, according to claim 6, **characterized** because the blunt tip of the bar is surrounded, during the application of the treatment, by a disc containing at least a permanent magnet.

8. Device to be used to impart therapeutic treatments with magnetic fields and magneto acupuncture, according to claim 7, **characterized** because at least one coil fed by one of the currents supplied by the generator can be used instead of one of the permanent magnets of the disc.
